# EUROPEAN PATENT SPECIFICATION

(11) **EP 3 914 105 B1**
(45) Date of publication and mention of the grant of the patent: **13.12.2023**
(21) Application number: 20702390.4
(22) Date of filing: 22.01.2020
(51) Int. Cl.: A24F 1/30, A24D 1/14, A24F 40/42, A61M 15/00

(54) **SHISHA CARTRIDGE WITH CAP**
SHISHA-KARTUSCHE MIT KAPPE
CARTOUCHE SHISHA AVEC CAPUCHON

(30) Priority: 25.01.2019 EP 19153850
(43) Date of publication of application: 01.12.2021
(73) Proprietor: Philip Morris Products S.A., 2000 Neuchâtel (CH)
(72) Inventor: NICOLAS, Frederic, 44000 Nantes (FR)
(74) Representative: Abitz & Partner
(86) International application number: PCT/IB2020/050503
(87) International publication number: WO 2020/152606

(56) References cited:
- EP-A1- 2 179 667
- EP-A1- 2 890 255
- EP-A1- 3 216 357
- EP-A2- 1 780 146
- EP-B1- 2 861 504
- WO-A1-2009/088521
- WO-A1-2016/082851
- JP-A- 2011 031 955
- US-A1- 2018 317 544

## Description

This disclosure relates to cartridges for housing an aerosol-forming substrate, particularly for use in aerosol-generating devices.

Traditional shisha devices are used to smoke tobacco and are configured such that vapor and smoke pass through a water basin before inhalation by a consumer. Shisha devices may include one outlet, or more than one outlet so that the device may be used by more than one consumer at a time. Use of shisha devices is considered by many to be a leisure activity and a social experience.

Typically, traditional shishas are used in combination with a substrate known as molasses that is relatively high in sugar (in some cases, up to -50% vs. the -20% typically found in tobacco substrate). The tobacco used in shisha devices may be mixed with other ingredients to, for example, increase the volume of the vapor and smoke produced, to alter flavor, or both.

Traditional shisha devices employ charcoal to combust the tobacco substrate to generate an aerosol for inhalation by a user. Using charcoal pellets to heat the tobacco may cause full or partial combustion of the tobacco or other ingredients. Additionally, charcoal pellets may generate harmful or potentially harmful products, such as carbon monoxide, which may mix with the shisha vapor and pass through the water basin to the outlet.

One way to reduce the production of carbon monoxide and combustion by-products is to employ e-liquids rather than tobacco. Shisha devices that employ e-liquids eliminate combustion by-products but deprive shisha consumers of the traditional tobacco-based experience.

Other shisha devices have been proposed that employ electric heaters to heat, but not combust, tobacco. Such electrically heated heat-not-burn shisha devices heat the tobacco substrate to a temperature sufficient to produce an aerosol from the substrate without combusting the substrate, and therefore reduce or eliminate by-products associated with combustion of tobacco.

Shisha devices may employ a cartridge for housing an aerosol-forming substrate. The cartridge may be filled with such aerosol-forming substrate. The aerosol-forming substrate may comprise tobacco, preferably shisha substrate, such as molasses-a mixture of tobacco, water, sugar, and other components, such as glycerine, flavors, etc. The heating system of the electrically heated shisha device heats the contents of the cartridge to generate aerosol, which is conveyed through an airflow path to a user.

In order to facilitate airflow through the cartridge and the flow of the aerosol from the cartridge, a shisha cartridge may have one or more holes through one or more walls. The cartridge may include one or more holes at the top, one or more holes at the bottom, or both one or more holes at the top and one or more holes at the bottom. The holes may also be disposed along the sides of the cartridge. Alternatively, the top may be open, that is, the top wall may be partially or completely absent. Any holes or openings in the top and bottom walls may be closed by a removable sealing layer, such as a film, sticker, or liner, during storage. The removable layer may protect the contents (for example, the molasses) from exposure to air and oxygen. The removable layer may be removed (for example, pulled or peeled off) by a user prior to first use of the cartridge.

The holes or openings in the cartridge, if left unsealed, can lead to loss of freshness (for example, moisture content) or contamination of the substrate, as well as issues with leakage. For one or more reasons, such as in order to maintain freshness, to prevent leakage of the substrate, or to preserve the quality and integrity of the substrate during storage, it is desirable to close or seal the openings or holes of the cartridge prior to use or between uses if the entire contents of the cartridge are not used at once.

Some users may wish to extend the use of a cartridge over two or more usage sessions, or may wish to pause a usage session before the contents of the cartridge are completely depleted. However, exposure to air may cause oxidation of the molasses within a relatively short period of time. Therefore, the molasses may deteriorate in an opened cartridge if the cartridge is not fully consumed within a short period of time. Further, a typical molasses includes liquid components that may leak from the cartridge if the cartridge is not fully consumed within a short period of time, even if stored inside the shisha device. Leaked molasses may contaminate the inside of the shisha device, and may be messy, unhygienic, or unpleasant for user, and compromise future sensorial experiences as reduced freshness.

Existing removable stickers or lids used to seal cartridges during storage may be attached by an adhesive, welding, crimping, or a combination thereof. However, such stickers or lids are typically disposable and are not replaceable or resealable to close the cartridge between uses.

EP 1 780 146 A2 discloses a dual functional dispenser. JP2011031955 A discloses a shake-out container. WO 2009/088521 A1 discloses a smokable material containment vessel designed for use with a hookah.

Furthermore, EP 2 179 667 A1 pertains to a hookah cartridge which comprises a container wall and which contains a portion of hookah tobacco within this container wall; wherein the container wall has perforations and wherein means are provided by which the perforations in the container wall are closed in a moisture-tight manner.

It would be desirable to provide a shisha cartridge that can provide multiple uses or that can be used a portion at a time. It would be desirable to provide a shisha cartridge that can be closed, preferably resealed, after use. It would further be desirable to provide a shisha cartridge that enables protection of remaining molasses (or other aerosol-forming substrate) in the cartridge after the cartridge has be unsealed, for example, if the entire aerosol-forming substrate has not been entirely depleted during a usage session. According to some aspects, it would be desirable to provide a cartridge with a body and a cap, where the body forms a bottom portion of the cartridge and the cap forms a top portion of the cartridge.

The present invention relates to a cartridge according to claim 1 and a shisha system according to claim 9.

Various aspects of the present disclosure relate to a shisha cartridge comprising a cap to close, seal, or re-seal an opening (for example, and opening in a top wall, bottom wall, or side wall; preferably a top wall) of the cartridge body. The body of the cartridge has one or more openings. The cap covers at least a portion of the opening. In some embodiments, the cap may have one or more openings. The opening of the body or the cap may include a single hole (for example, an open top, or an opening in a top wall) or a plurality of holes. The plurality of holes may comprise perforations through the material, a mesh, or an air-permeable material.

According to another aspect of the present disclosure, the shisha cartridge comprises a cap that includes openings. Prior to use, the openings may be closed (for example sealed) by a removable (for example, peelable) film, sticker, or liner.

According to some aspects, the cap may be removable. The cap may be rotatable about an axis. According to some aspects, the cap may include a lid portion and a cylindrical portion that extends axially from the lid portion.

According to some aspects, the cap of the present disclosure allows the user to easily open and close the cartridge. For example, the user may temporarily close the cartridge between uses. The cap may allow the user to use only a portion of the aerosol-forming substrate without risking contamination of the contents or of the shisha device. According to another aspect, the cap of the present disclosure includes openings providing an airflow path from the cartridge to the outside of the cartridge.

One aspect of the present disclosure relates to a shisha cartridge for housing an aerosol-forming substrate, the cartridge comprising a body comprising one or more walls, and a wall opening in at least one of the one or more walls; a cavity in the body in fluid communication with the at least one wall opening; and a cap comprising a cap opening hingedly closed by a hatch; and a removable layer removably attached to the body and disposed between the body and the cap.

The term "aerosol" is used here to refer to a suspension of fine solid particles or liquid droplets in a gas, such as air, which may contain volatile flavor compounds.

The term "substrate" is used here to refer to a consumable material that may be disposed inside the cartridge.

The terms "integral" and "integrally formed" are used here to describe elements that are formed in one piece (a single, unitary piece) and cannot be separably removed from each other without causing structural damage to the piece.

As used herein, the singular forms "a," "an," and "the" encompass embodiments having plural referents, unless the content clearly dictates otherwise.

As used herein, "or" is generally employed in its sense including "one or the other or both" unless the content clearly dictates otherwise.

As used herein, "have," "having," "include," "including," "comprise," "comprising" or the like are used in their open-ended sense, and generally mean "including, but not limited to". It will be understood that "consisting essentially of," "consisting of," and the like are subsumed in "comprising," and the like.

The words "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the disclosure, including the claims.

The term "substantially" as used here has the same meaning as "significantly," and can be understood to modify the term that follows by at least about 90 %, at least about 95 %, or at least about 98 %. The term "not substantially" as used here has the same meaning as "not significantly," and can be understood to have the inverse meaning of "substantially," i.e., modifying the term that follows by not more than 10 %, not more than 5 %, or not more than 2 %.

Any direction referred to herein, such as "top," "bottom," "left," "right," "upper," "lower," and other directions or orientations are described herein for clarity and brevity are not intended to be limiting of an actual device or system. Devices and systems described herein may be used in a number of directions and orientations.

The cartridge may comprise any suitable body defining a cavity. Aerosol-forming substrate may be disposed in the cavity of the cartridge. The body is preferably formed from one or more heat resistant materials, such as a heat resistant metal or polymer. The body may comprise a thermally conductive material. For example, the body may comprise any of: aluminum, copper, zinc, nickel, silver, any alloys thereof, and combinations thereof. Preferably, the body comprises aluminum.

The cartridge may be of any suitable shape. For example, the cartridge may have a shape configured to be received by a shisha device. The cartridge may have a substantially cuboidal shape, cylindrical shape, frustoconical shape, or any other suitable shape. Preferably, the cartridge has a generally cylindrical shape or a frustoconical shape.

The shisha device is configured to heat the aerosol-forming substrate in the cartridge. The device may be configured to heat the aerosol-forming substrate in the cartridge by conduction. The cartridge is preferably shaped and sized to allow contact with, or minimize distance from, a heating element of the shisha device to provide efficient heat transfer from the heating element to the aerosol-forming substrate in the cartridge. The heat may be generated by any suitable mechanism, such as by resistive heating or by induction. In order to facilitate inductive heating, the cartridge may be provided with a susceptor. For example, the cartridge body may be made from or include a material (for example, aluminum) that is capable of acting as a susceptor, or a susceptor material may be provided within the cavity of the cartridge. A susceptor material may be provided within the cavity of the cartridge in any form, for example a powder, a solid block, shreds, etc.

Any suitable aerosol-forming substrate may be provided in the cavity defined by the body of the cartridge. The aerosol-forming substrate is preferably a substrate capable of releasing volatile compounds. The aerosol-forming substrate is preferably a substrate capable of releasing compounds that may form an aerosol. The volatile compounds may be released by heating the aerosol-forming substrate. The aerosol-forming substrate may be solid or liquid or comprise both solid and liquid components. Preferably, the aerosol-forming substrate is a shisha substrate. A shisha substrate is understood to mean a consumable material that is suitable for use in a shisha device. Shisha substrate may include molasses.

The aerosol-forming substrate may include nicotine. The nicotine containing aerosol-forming substrate may include a nicotine salt matrix. The aerosol-forming substrate may include plant-based material. The aerosol-forming substrate preferably includes tobacco. The tobacco containing material preferably contains volatile tobacco flavor compounds, which are released from the aerosol-forming substrate upon heating. The aerosol-forming substrate may include homogenized tobacco material. Homogenized tobacco material may be formed by agglomerating particulate tobacco. The aerosol-forming substrate may alternatively or additionally include a non-tobacco-containing material. The aerosol-forming substrate may include homogenized plant-based material.

The aerosol-forming substrate may include, for example, one or more of: powder, granules, pellets, shreds, spaghettis, strips, or sheets. The aerosol-forming substrate may contain one or more of: herb leaf, tobacco leaf, fragments of tobacco ribs, reconstituted tobacco, homogenized tobacco, extruded tobacco, and expanded tobacco.

The aerosol-forming substrate may include at least one aerosol former. Suitable aerosol formers include compounds or mixtures of compounds which, in use, facilitate formation of a dense and stable aerosol and which are substantially resistant to thermal degradation at the operating temperature of the shisha device. Suitable aerosol formers are well known in the art and include, but are not limited to: polyhydric alcohols, such as triethylene glycol, 1,3-butanediol and glycerine; esters of polyhydric alcohols, such as glycerol mono-, di- or triacetate; and aliphatic esters of mono-, di- or polycarboxylic acids, such as dimethyl dodecanedioate and dimethyl tetradecanedioate. Particularly preferred aerosol formers are polyhydric alcohols or mixtures thereof, such as triethylene glycol, 1,3-butanediol and, most preferred, glycerine. The aerosol-forming substrate may include any suitable amount of an aerosol former. For example, the aerosol former content of the substrate may be equal to or greater than 5 % on a dry weight basis, and preferably greater than 30 % by weight on a dry weight basis. The aerosol former content may be less than about 95 % on a dry weight basis. Preferably, the aerosol former content is up to about 55 %.

The aerosol-forming substrate preferably includes nicotine and at least one aerosol former. In some embodiments, the aerosol former is glycerine or a mixture of glycerine and one or more other suitable aerosol formers, such as those listed above.

The aerosol-forming substrate may include other additives and ingredients, such as flavorants, sweeteners, etc. In some examples, the aerosol-forming substrate includes one or more sugars in any suitable amount. Preferably, the aerosol-forming substrate includes invert sugar, which is a mixture of glucose and fructose obtained by splitting sucrose. Preferably, the aerosol-forming substrate includes from about 1 % to about 40 % sugar, such as invert sugar, by weight. In some example, one or more sugars may be mixed with a suitable carrier such as cornstarch or maltodextrin.

In some examples, the aerosol-forming substrate includes one or more sensory-enhancing agents. Suitable sensory-enhancing agents include flavorants and sensation agents, such as cooling agents. Suitable flavorants include natural or synthetic menthol, peppermint, spearmint, coffee, tea, spices (such as cinnamon, clove, ginger, or combination thereof), cocoa, vanilla, fruit flavors, chocolate, eucalyptus, geranium, eugenol, agave, juniper, anethole, linalool, and any combination thereof.

In some examples, the aerosol-forming substrate is in the form of a suspension. For example, the aerosol-forming substrate may include molasses. As used herein, "molasses" means an aerosol-forming substrate composition comprising about 20 % or more sugar. For example, the molasses may include at least about 25 % by weight sugar, such as at least about 35 % by weight sugar. Typically, the molasses will contain less than about 60 % by weight sugar, such as less than about 50 % by weight sugar.

Any suitable amount of aerosol-forming substrate (for example, molasses or tobacco substrate) may be disposed in the cavity. In some preferred embodiments, about 3 g to about 25 g of the aerosol-forming substrate is disposed in the cavity. The cartridge may include at least 6 g, at least 7 g, at least 8 g, or at least 9 g of aerosol-forming substrate. The cartridge may include up to 15 g, up to 12 g; up to 11 g, or up to 10 g of aerosol-forming substrate. Preferably, from about 7 g to about 13 g of aerosol-forming substrate is disposed in the cavity.

The body of the cartridge may include one or more walls. In some embodiments, the body includes a top wall, a bottom wall, and a sidewall. In some embodiments, the top of the body is open (that is, the body does not include a top wall). The sidewall may be cylindrical or frustoconical, extending from the bottom to the top. In some embodiments, the sidewall includes a frustoconical portion (for example the bottom portion) and a cylindrical portion (for example the top portion). The body may include one or more parts. For example, the sidewall and the bottom wall may be an integral single part. The sidewall and the bottom wall may be two parts configured to engage one another in any suitable manner. For example, the sidewall and the bottom wall may be configured to engage one another by threaded engagement or interference fit. The sidewall and the bottom wall may be two parts joined together. For example, the sidewall and the bottom wall may be joined together by welding or by an adhesive. The top wall and sidewall may be a single integral part. The sidewall and the top wall may be two parts configured to engage one another in any suitable manner. For example, sidewall and the top wall may be configured to engage one another by threaded engagement or interference fit. The sidewall and the top wall may be two parts joined together. For example, the sidewall and the top wall may be joined together by welding or by an adhesive. The top wall, sidewall and bottom wall may all be a single integral part. The top wall, the sidewall, and the bottom wall may be three separate parts configured to engage one another in any suitable manner. For example, the top wall, the sidewall, and the bottom wall may be configured to engage by threaded engagement interference fit, welding, or an adhesive.

One or more walls of the body may form a heatable wall or surface. As used here, "heatable wall" and "heatable surface" mean an area of a wall or a surface to which heat may be applied, either directly or indirectly. The heatable wall or surface may function as a heat transfer surface through which heat may be transferred from outside of the body to the cavity or to an internal surface of the cavity.

The body of the cartridge may have one or more interior walls dividing the cavity into compartments. For example, the cavity may be divided into a first compartment and a second compartment. The cavity may be divided into 2, 3, 4, 5, 6, 7, or even 8 compartments. The interior wall may extend from the bottom of the body to the top. In some embodiments, the interior wall may have perforations or openings.

Preferably, the body of the cartridge has a length (for example, an axial length along a vertical center axis) of about 15 cm or less. In one embodiment, the body has a length of about 10 cm or less. The body may have an inside diameter of about 1 cm or more. The inside diameter of the body may be about 1.75 cm or more. The cartridge may have a heatable surface area in the cavity from about 25 cm² to about 100 cm², such as from about 70 cm² to about 100 cm². The volume of the cavity may be from about 10 cm³ to about 50 cm³; preferably from about 25 cm³ to about 40 cm³. In one embodiment, the body has a length in a range from about 3.5 cm to about 7 cm. The inside diameter of the body may be from about 1.5 cm to about 4 cm. The body may have a heatable surface area in the cavity from about 30 cm² to about 100 cm², such as from about 70 cm² to about 100 cm². The volume of the cavity may be from about 10 cm³ to about 50 cm³; preferably from about 25 cm³ to about 40 cm³. Preferably, the body is cylindrical or frustoconical.

The cartridge body may include one or more openings or ventilation holes through one or more walls of the body. The ventilation holes may be inlets, outlets, or both. The ventilation holes may be disposed at the bottom wall, top wall, sides, or a combination thereof, of the cartridge. In some embodiments, the cartridge includes one or more inlets and one or more outlets to allow air to flow through the aerosol-forming substrate when the cartridge is used with a shisha device. In some embodiments, the top wall of the cartridge may be absent or may define one or more openings to form the one or more inlets of the cartridge. The bottom wall of the cartridge may define one or more openings to form the one or more outlets of the cartridge. Preferably, the one or more inlets and outlets are sized and shaped to provide a suitable resistance to draw (RTD) through the cartridge. In some examples, the RTD through the cartridge, from the inlet or inlets to the outlet or outlets, may be from about 10 mm H₂O to about 50 mm H₂O, preferably from about 20 mm H₂O to about 40 mm H₂O. The RTD of a specimen refers to the static pressure difference between the two ends of the specimen when it is traversed by an air flow under steady conditions in which the volumetric flow is 17.5 milliliters per second at the output end. The RTD of a specimen may be measured using the method set out in ISO Standard 6565:2002.

According to one embodiment, the cartridge includes a cap connected to the body. The cap may be used to close and re-close openings on the cartridge body. Alternatively, the cap may form a removable top portion of the body and may include openings.

The cap may be rotatable about a center axis that extends through the cap. The center axis of the cap may be parallel or coaxial with a center axis of the cartridge body. The center axis of the cartridge body is considered to be an imaginary line that passes vertically through the center of the body when the body is in an upright position. The cap is preferably removable. The cap may be attached to the body by any suitable mechanism, such as a fastener, snap fit, threaded fit, bayonet connection, friction fit or by a magnetic connection.

The cap may at least partially cover a top wall (or an open top) or a bottom wall of the body. The cap may cover at least a portion of a side wall of the body. The cap may have a lid portion that covers the top or bottom of the body of the cartridge. The lid portion may be planar or may have a contour (for example, be domed). The lid portion may form the top surface of the cap. The cap may also include a cylindrical portion extending axially in the direction of the center axis from the lid portion. The cylindrical portion may be a side wall of the cap and may cover at least a portion of a side wall of the body. In some embodiments, the cap only includes a lid portion and does not include a cylindrical portion. The cylindrical portion of the cap may be constructed to fit over or mate with a top portion (for example a cylindrical portion) of the cartridge body.

In some embodiments, the cap may have one or more cap openings. For example, the cap may have a cap opening in the lid portion, in the cylindrical portion, or both. The cap opening may be a hole or aperture through the material of the cap, or may be a cut-out extending to the edge of the cap, or both. The cap opening may include a single hole or a plurality of holes. The cap opening may be further closed by a hatch. The hatch may be hingedly attached to the cap.

The cap opening may have any suitable size and shape. For example, the cap opening may be round, polygonal (for example, square, triangle, etc.), sector-shaped, a slot, or have an irregular shape. The size of the cap opening may be expressed as a percentage of the outside area of the cap. The outside area of the cap is understood to mean the surface area of the cap facing outward (for example, not facing the body), such as the outward-facing surface area of the lid portion and the cylindrical portion. In some embodiments, the cap opening may comprise 5 % or greater, 10 % or greater, 15 % or greater, 20 % or greater, or 25 % or greater of the area of the cap. The cap opening may comprise 75 % or less, 50 % or less, 40 % or less, or 30 % or less of the area of the cap.

A cap with a cap opening may be combined with a body having an open top (that is, having no top wall), or a body having a body opening in the top wall that may be aligned with the cap opening.

The openings of the body and cap do not necessarily have to be the same shape, number, or size. In some embodiments, the cap opening is different (for example, has a different shape, size, or number of holes) from the body opening. In some embodiments, the cap opening is the same as or similar to (for example, has the same shape, size, or number of holes) the body opening.

The openings of the body and cap may be positioned such that by rotating the cap to a first position, at least some of the openings are aligned such that the cavity of the body is in fluid communication with the environment (for example, with the airflow path of the shisha device) through the aligned openings, and by rotating the cap to a second position, the openings are not aligned (that is, the cap closes the openings of the body).

The cartridge may further include a seal or layer covering the one or more inlets and optionally a second seal or layer covering the one or more outlets prior to use. The seal is preferably removable. The removable layer may be disposed between the body and the cap, or on the outside of the cap. The removable layer may substantially or completely cover the lid portion of the cap. The removable layer may cover the lid portion of the cap from the inside or from the outside. The removable layer may include a peelable label, sticker, foil, or the like. The label, sticker, or foil may be affixed to the cartridge in any suitable manner, such as with an adhesive, crimping, welding, or otherwise being joined to the container. The removable layer may include a tab that may be grasped to peel or remove the label, sticker, or foil from the cartridge prior to first use. In some embodiments, the user may first remove the cap and then peel or remove the removable layer. Depending on the type of cap, the user may replace the cap on the body prior to use (for example, when the cap includes a cap opening) or after use (for example, when the cap does not include a cap opening).

The cartridge may further include features that facilitate sealing or re-sealing the cartridge. For example, the cartridge body, the cap, or both may include a sealing member, such as an O-ring or a liner, made from a resilient material, such as rubber. In one embodiment, the cartridge body, the cap, or both include a sealing member to facilitate a seal between the cartridge body and the cap.

In some embodiments the cartridge is a shisha cartridge that may be used with any suitable shisha device. Preferably, the shisha device is configured to sufficiently heat the aerosol-forming substrate in the cartridge to form an aerosol from the aerosol-forming substrate but not to combust the aerosol-forming substrate. For example, the shisha device may be configured to heat the aerosol-forming substrate to a temperature in a range from about 150 °C to about 300 °C; more preferably from about 180 °C to about 250 °C or from about 200 °C to about 230 °C.

The shisha device may include a receptacle for receiving the cartridge. The shisha device may include a heating element configured to contact or to be in proximity to the body of the cartridge when the cartridge is received in the receptacle. The heating element may form at least part of the receptacle. For example, the heating element may form at least a portion of the surface of the receptacle. The shisha cartridge may be configured to transfer heat from the heating element to the aerosol-forming substrate in the cavity by conduction. In some embodiments, the heating element includes an electric heating element. In some embodiments, the heating element includes a resistive heating component. For example, the heating element may include one or more resistive wires or other resistive elements. The resistive wires may be in contact with a thermally conductive material to distribute heat produced over a broader area. Examples of suitable conductive materials include aluminum, copper, zinc, nickel, silver, and combinations thereof. The heating element may form at least a portion of the surface of the receptacle.

The shisha device may include control electronics operably coupled to the heating element. The control electronics may be configured to control heating of the heating element. The control electronics may be configured to control the temperature to which the aerosol-forming substrate in the cartridge is heated. The control electronics may be provided in any suitable form and may, for example, include a controller or a memory and a controller. The controller may include one or more of an Application Specific Integrated Circuit (ASIC) state machine, a digital signal processor, a gate array, a microprocessor, or equivalent discrete or integrated logic circuitry. Control electronics may include memory that contains instructions that cause one or more components of the circuitry to carry out a function or aspect of the control electronics. Functions attributable to control electronics in this disclosure may be embodied as one or more of software, firmware, and hardware.

The electronic circuitry may include a microprocessor, which may be a programmable microprocessor. The electronic circuitry may be configured to regulate a supply of power. The power may be supplied to the heater element in the form of pulses of electrical current.

In some examples, the control electronics may be configured to monitor the electrical resistance of the heating element and to control the supply of power to the heating element depending on the electrical resistance of the heating element. In this manner, the control electronics may regulate the temperature of the resistive element.

The shisha device may include a temperature sensor, such as a thermocouple. The temperature sensor may be operably coupled to the control electronics to control the temperature of the heating element. The temperature sensor may be positioned in any suitable location. For example, the temperature sensor may be configured to insert into the cartridge when received within the receptacle to monitor the temperature of the aerosol-forming substrate being heated. In addition or alternatively, the temperature sensor may be in contact with the heating element. In addition or alternatively, the temperature sensor may be positioned to detect temperature at an aerosol outlet of the shisha device or a portion thereof. The sensor may transmit signals regarding the sensed temperature to the control electronics. The control electronics may adjust heating of the heating elements in response to the signal to achieve a suitable temperature at the sensor.

The control electronics may be operably coupled to a power supply. The shisha device may include any suitable power supply. For example, a power supply of a shisha device may be a battery or set of batteries. The batteries of the power supply may be rechargeable, removable and replaceable, or rechargeable and removable and replaceable. Any suitable battery may be used. For example, heavy duty type or standard batteries existing in the market, such as used for industrial heavy duty electrical power-tools. Alternatively, the power supply may be any type of electric power supply including a super or hyper-capacitor. Alternatively, the assembly may be connected to an external electrical power source, and electrically and electronically designed for such purpose. Regardless of the type of power supply employed, the power supply preferably provides sufficient energy for the normal functioning of the assembly for at least one shisha session until aerosol is depleted from the aerosol-forming substrate in the cartridge before being recharged or needing to connect to an external electrical power source. Preferably, the power supply provides sufficient energy for the normal functioning of the assembly for at least about 70 minutes of continuous operation of the device, before being recharged or needing to connect to an external electrical power source.

In one example, a shisha device includes an aerosol-generating element that includes a cartridge receptacle, a heating element, an aerosol outlet, and a fresh air inlet. The cartridge receptacle is configured to receive a cartridge according to the present disclosure containing the aerosol-forming substrate. The heating element may define at least part of a surface of the receptacle.

The shisha device includes a fresh air inlet channel in fluid connection with the receptacle. In use, when the substrate inside the cartridge is heated, aerosol former components in the substrate vaporize. Air flowing from the fresh air inlet channel through the cartridge becomes entrained with aerosol generated from the aerosol former components in the cartridge.

Some electrically heated shisha devices employ pre-heated air and typically employ an airflow path such that the air travels in the vicinity of the heat source upon puffing. Further, some electrically heated shisha devices employ elements that increase radiation heat transfer by increasing the heated surface area.

The fresh air inlet channel may include one or more apertures through the cartridge receptacle such that fresh air from outside the shisha device may flow through the channel and into the cartridge receptacle through the one or more apertures. If a channel includes more than one aperture, the channel may include a manifold to direct air flowing through the channel to each aperture. Preferably, the shisha device includes two or more fresh air inlet channels.

As described above, the cartridge includes one or more openings (such as inlets or outlets) formed in the body. A cap with a cap opening may be placed on the body, allowing air to flow through the cartridge. Alternatively, a cap without a cap opening may be removed to allow air to flow through the cartridge. If the receptacle includes one or more inlet apertures, at least some of the inlets in the cartridge may align with the apertures in the top of the receptacle. The cartridge may include an alignment feature configured to mate with a complementary alignment feature of the receptacle to align the inlets of the cartridge with the apertures of the receptacle when the cartridge is inserted into the receptacle.

Air that enters the cartridge may flow across or through, or both across and through the aerosol-forming substrate, entraining aerosol, and exiting the cartridge and receptacle via an aerosol outlet. From the aerosol outlet, the air carrying the aerosol enters a vessel of the shisha device.

The shisha device may include any suitable vessel defining an interior volume configured to contain a liquid and defining an outlet in the head-space above a liquid fill level. The vessel may include an optically transparent or opaque housing to allow a consumer to observe contents contained in the vessel. The vessel may include a liquid fill demarcation, such as a liquid fill line. The vessel housing may be formed of any suitable material. For example, the vessel housing may include glass or suitable rigid plastic material. Preferably, the vessel is removable from a portion of the shisha assembly comprising the aerosol-generation element to allow a consumer to fill, empty or clean the vessel.

The vessel may be filled to a liquid fill level by a consumer. The liquid preferably includes water, which may optionally be infused with one or more colorants, flavorants, or colorants and flavorants. For example, the water may be infused with one or both of botanical or herbal infusions.

Aerosol entrained in air exiting the aerosol outlet of the receptacle may travel through a conduit positioned in the vessel. The conduit may be coupled to the aerosol outlet of the aerosol-generating element and may have an opening below the liquid fill level of the vessel, such that aerosol flowing through the vessel flows through the opening of the conduit, then through the liquid, into headspace of the vessel and exits through a headspace outlet, for delivery to a consumer.

The headspace outlet may be coupled to a hose comprising a mouthpiece for delivering the aerosol to a consumer. The mouthpiece may include an activation element, such as a switch activatable by a user, a puff sensor arranged to detect a user puffing on the mouthpiece, or both a switch activatable by the user and a puff sensor. The activation element is operably coupled to the control electronics of the shisha device. The activation element may be wirelessly coupled to the control electronics. Activation of the activation element may cause the control electronics to activate the heating element, rather than constantly supplying energy to the heating element. Activation of an activation element may cause the control electronics to activate the heating element, rather than constantly supplying energy to the heating element. Accordingly, the use of an activation element may serve to save energy relative to devices not employing such elements to provide on-demand heating rather than constant heating.

For purposes of example, one method for using a shisha device as described herein is provided below in chronological order. The vessel may be detached from other components of the shisha device and filled with water. One or more of natural fruit juices, botanicals, and herbal infusions may be added to the water for flavoring. The amount of liquid added should cover a portion of the conduit but should not exceed a fill level mark that may optionally exist on the vessel. The vessel is then reassembled to the shisha device. The cartridge may be prepared by removing any removable layer (if present). If the cap includes an opening, the cap may be replaced on the cartridge, optionally rotating the cap so that the cap opening aligns with an opening on the body. If the cap does not include an opening, the cap may be removed for duration of the use of the cartridge. A portion of the aerosol-generating element may be removed or opened to allow the cartridge to be inserted into the receptacle. The aerosol-generating element is then reassembled or closed. The device may then be turned on. Turning on the device may initiate a heating profile of a heating element, to heat the aerosol-forming substrate to a temperature at or above a vaporization temperature but below a combustion temperature of the aerosol-forming substrate. The aerosol forming compounds of the aerosol-forming substrate vaporize, generating an aerosol. The user may puff on the mouth piece as desired. The user may continue using the device as long as desired or until no more aerosol is visible or being delivered. In some embodiments, the device may be arranged to automatically shut off when the cartridge or a compartment of the cartridge is depleted of usable aerosol-forming substrate. The user may replace the cap on the cartridge or rotate the cap on the cartridge to close the cartridge. In some embodiments, the consumer may refill the device with a fresh cartridge after, for example, receiving the cue from the device that the aerosol-forming substrate in the cartridge is depleted or nearly depleted. The shisha device may be turned off at any time by a consumer by, for example, switching off the device.

The shisha device may have any suitable air management. In one example, puffing action from the user will create a suction effect causing a low pressure inside the device which will cause external air to flow through an air inlet of the device, into the fresh air inlet channel, and into the receptacle. The air may then flow through the cartridge in the receptacle to carry aerosol produced from the aerosol-forming substrate. The air with entrained aerosol then exits the aerosol outlet of the receptacle, flows through the conduit to the liquid inside the vessel. The aerosol will then bubble out of the liquid and into head space in the vessel above the level of the liquid, out the headspace outlet, and through the hose and mouthpiece for delivery to the consumer. The flow of external air and the flow of the aerosol inside the shisha device may be driven by the action of puffing from the user.

Reference will now be made to the drawings, which depict one or more aspects described in this disclosure. However, it will be understood that other aspects not depicted in the drawings fall within the scope and spirit of this disclosure. Like numbers used in the figures refer to like components. However, it will be understood that the use of a number to refer to a component in a given figure is not intended to limit the component in another figure labeled with the same number. In addition, the use of different numbers to refer to components in different figures is not intended to indicate that the different numbered components cannot be the same or similar to other numbered components. The figures are presented for purposes of illustration and not limitation. Schematic drawings presented in the figures are not necessarily to scale.
**FIG. 1** is a schematic view of a shisha device.
**FIGS. 2A** and **2B** are cross sectional views of a shisha cartridge with a cap and a removable layer for use in the shisha device of **FIG.** 1 according to an embodiment.
**FIG. 2C** is a cross sectional view of the shisha cartridge of **FIGS. 2A** and **2B** without the removable layer.
**FIGS. 3A-3D** are cross sectional views of a shisha cartridge with a cap and a removable layer for use in the shisha device of **FIG. 1** according to an embodiment.
**FIGS. 4A-4D** are views of the cap for a shisha cartridge for use in the shisha device of **FIG. 1** according to embodiments.
**FIGS. 5A** and **5B** are views of the body of a shisha cartridge for use in the shisha device of **FIG. 1** according to an embodiment.
**FIGS. 6A** and **6B** are cross-sectional views of attachment mechanisms of the cap to the body of the cartridge according to embodiments.
**FIG. 7A** is a perspective view of a shisha cartridge for use in the shisha device of **FIG. 1** according to an embodiment.
**FIG. 7B** is a cross-sectional view of the shisha cartridge of **FIG. 7A** with a lid according to an embodiment.

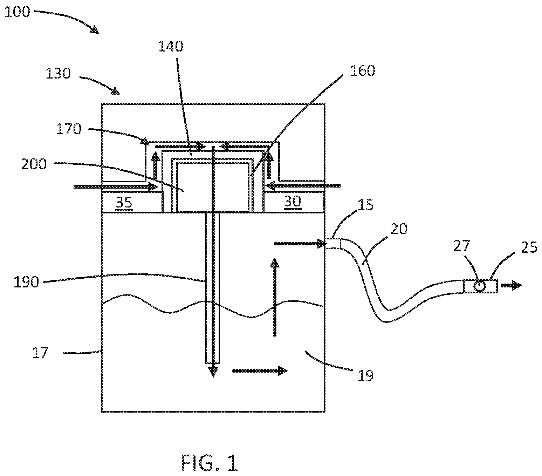

**FIG. 1** is a schematic sectional view of an example of a shisha device **100.** The device **100** includes a vessel **17** defining an interior volume configured to contain liquid **19** and defining a headspace outlet **15** above a fill level for the liquid **19.** The liquid **19** preferably includes water, which may optionally be infused with one or more colorants, one or more flavorants, or one or more colorants and one or more flavorants. For example, the water may be infused with one or both of botanical infusions or herbal infusions.

The device **100** also includes an aerosol-generating element **130.** The aerosol-generating element **130** includes a receptacle **140** configured to receive a cartridge **200** containing an aerosol-forming substrate. The aerosol-generating element **130** may also include a heating element **160** that forms at least one surface of the receptacle **140.** In the depicted embodiment, the heating element **160** defines the side surfaces and the top surface of the receptacle **140.** The aerosol-generating element **130** also includes a fresh air inlet channel **170** that draws fresh air into the device **100.** In some embodiments, portion of the fresh air inlet channel **170** is formed by the heating element **160** to heat the air before the air enters the receptacle **140.** The pre-heated air then enters the cartridge **200,** which is also heated by heating element **160,** to carry aerosol generated by the aerosol former and the aerosol-forming substrate. The air exits an outlet of the aerosol-generating element **130** and enters a conduit **190.**

The conduit **190** carries the air and aerosol into the vessel **17** below the level of the liquid **19.** The air and aerosol may bubble through the liquid **19** and exit the headspace outlet **15** of the vessel **17.** A hose **20** may be attached to the headspace outlet **15** to carry the aerosol to the mouth of a user. A mouthpiece **25** may be attached to, or form a part of, the hose **20.**

An exemplary air flow path of the device, in use, is depicted by thick arrows in **FIG. 1****.**

The mouthpiece **25** may include an activation element **27.** The activation element **27** may be a switch, button or the like, or may be a puff sensor or the like. The activation element **27** may be placed at any other suitable location of the device **100.** The activation element **27** may be in wireless communication with the control electronics **30** to place the device **100** in condition for use or to cause control electronics to activate the heating element **160;** for example, by causing power supply **35** to energize the heating element **160.**

The control electronics **30** and power supply **35** may be located in any suitable position of the aerosol-generating element **130,** including locations other than the bottom portion of the element **130** as depicted in **FIG. 1****.**

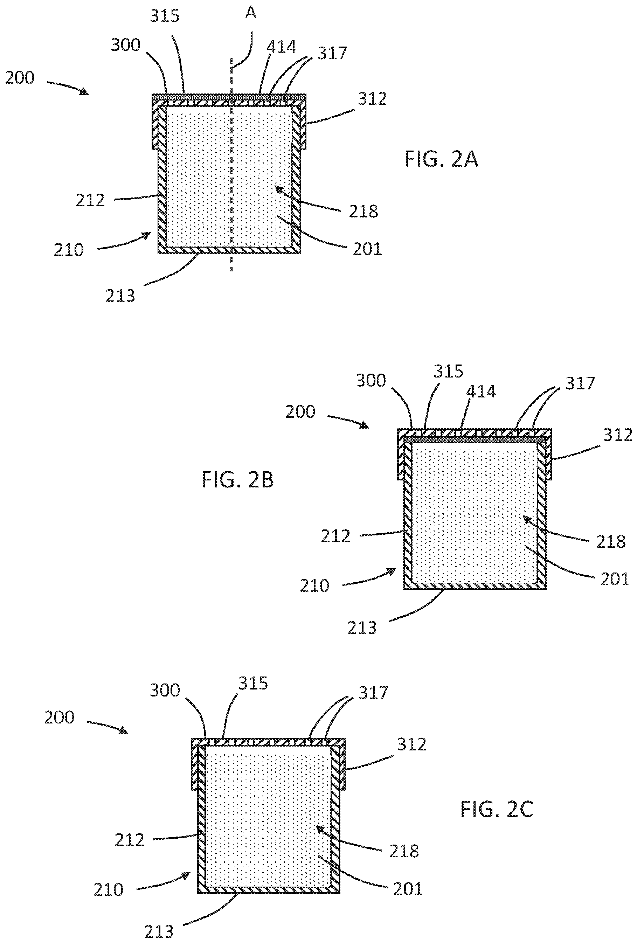

Referring to **FIGS. 2A-2C****,** a cartridge **200** has body **210** forming a cavity **218** in which an aerosol-forming substrate **201** may be disposed. The body **210** includes a top wall **215,** bottom wall **213,** and a sidewall **212.** The sidewall **212** may be cylindrical or frustoconical, or a combination of shapes. The body **210** may be formed from one or more parts. For example, the top wall **215** or bottom wall **213** may be removably attached to the sidewall **212** to allow the aerosol-forming substrate **201** to be disposed in the cavity **218.** The cartridge **200** also includes a cap **300.** In the example shown, the cap **300** at least partially covers the top wall **215** and a part of the side wall **212.**

In the example shown in **FIG. 2A****,** the cap **300** includes a lid portion **315** and a cylindrical portion **312** extending axially from the lid portion **315.** The size (depth) of the cylindrical portion **312** may vary. The cap **300** may alternatively be provided with the lid portion **315** only, without a cylindrical portion **312.** The lid portion **315** may at least partially cover the open top of the body **210.** The lid portion **315** may be a planar portion. The cap **300** has a cap opening **317** extending through the lid portion **315.** In the example shown in **FIG. 2A****,** the cap opening **317** includes a plurality of through holes. The cap **300** may be rotatable about the center axis **A.** If the body **210** includes a top wall **215** with an opening, rotating the cap **300** may be used to open the cartridge **200** by aligning the cap opening **317** with an opening on the body **210.**

Prior to first use, the cartridge **200** may include a removable layer **414.** The removable layer **414** may be disposed on top of the cap **300,** as shown in **FIG. 2A****,** or underneath the cap **300,** as shown in **FIG. 2B****.** A user may remove the removable layer **414** prior to use and replace the cap **300** (if necessary) on the cartridge body **210.** In some embodiments, the cartridge **200** may be provided with a second cap that has a top surface without openings or holes, and a user may replace the cap **300** with the second cap (or place the second cap on top of cap **300**) to close or preferably seal the cartridge **200.**

**FIG. 2C** shows the cartridge **200** after the removable layer **414** has been removed. For example, in the example where the removable layer **414** is underneath the cap **300,** after the removable layer **414** is removed, the cap **300** may be re-positioned on the cartridge body **210** as shown in **FIG. 2C****.** The cartridge **200** is ready for use, allowing air to flow from openings at the bottom **213** (not shown) through the cavity **218** and through the openings **317** of the cap **300.**

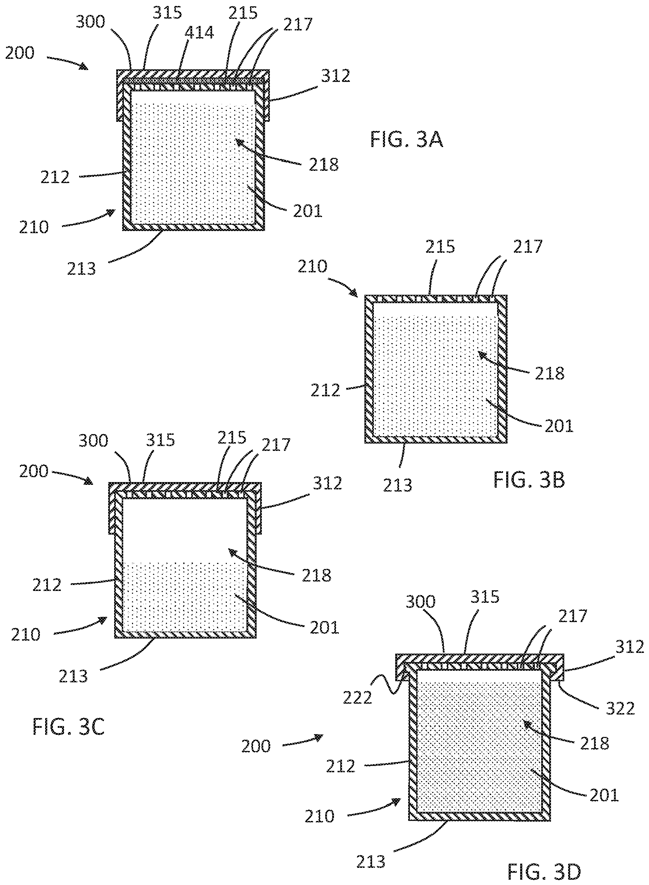

In the examples shown in **FIGS. 3A** and **3D****,** the cap **300** does not have any openings or holes. The cap **300** may otherwise be similar to the cap shown in **FIGS. 2A-2C****,** including a lid portion **315** and a cylindrical portion **312** extending axially from the lid portion **315.** The body **210** may include a top wall **215** with an opening **217.** In the example shown, the cap opening **217** includes a plurality of through holes extending through the top wall **215.** **FIG. 3D** shows an alternative cap having a snap fit attachment with a lip **222** on the body **210** and a corresponding lip **322** on the cap **300** that holds the cap **300** in place.

Prior to first use, the cartridge **200** may include a removable layer **414** attached to the top wall **215** of the body **210.** A user may remove the cap **300** and the removable layer **414** prior to use to allow airflow through the openings **317** of the cap. **FIG. 3B** illustrates the cartridge body **210** with the lid and the removable layer **414** removed, allowing airflow from openings at the bottom **213** (not shown) through the cavity **218** and through the openings **217** at the top wall **215** of the body **210.** After complete or partial use of the cartridge **200** (for example, in some instances, the user might only have used a portion of the aerosol-forming substrate **201** and not completed a usage session or completely depleted the aerosol-forming substrate **201**), the user may replace the cap **300** on the cartridge body **210** to close or preferably seal the cartridge **200,** as shown in **FIG. 3C****.** The cartridge body **210,** the cap **300,** or both may include a sealing member, such as an O-ring or a liner, made from a resilient material to facilitate a seal between the cartridge body **210** and the cap **300** after the removable layer **414** has been removed.

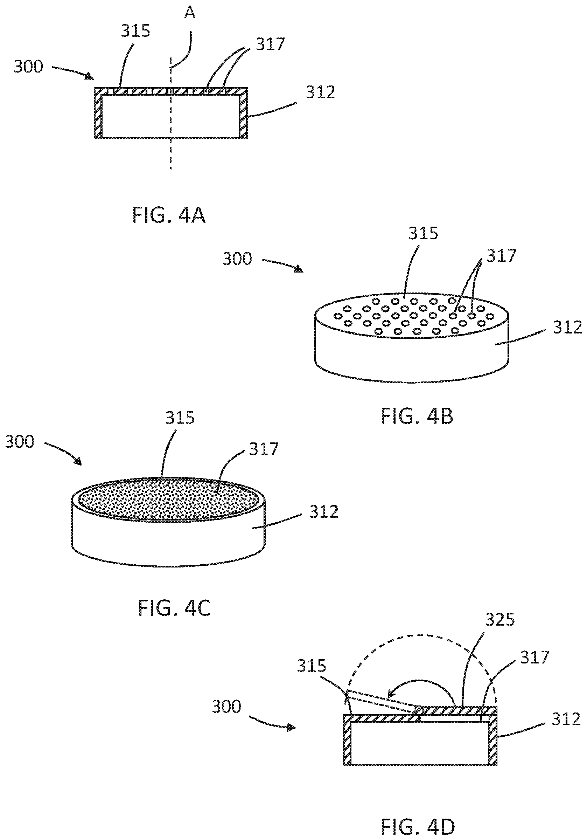

**FIGS. 4A-4C** demonstrate various embodiments of the cap **300.** The cap may include a lid portion **315,** which may be planar. The cap **300** may also include a cylindrical portion **312** extending axially (that is, in the direction of the center axis A**)** from the lid portion **315.** The cylindrical portion **312** may form a side wall of the cap. In some embodiments, the cap **300** includes a cap opening **317.** The cap opening **317** may include a single opening **317** or a plurality of openings **317** as shown in **FIG. 4A****.** In some embodiments, the cap opening **317** may be covered by a mesh, as shown in **FIG. 4C****.** In some embodiments, the cap **300** includes a hatch **325.** The hatch **325** may cover the cap opening **317,** as shown in **FIG. 4D****.** The hatch **325** may be hinged for opening and closing.

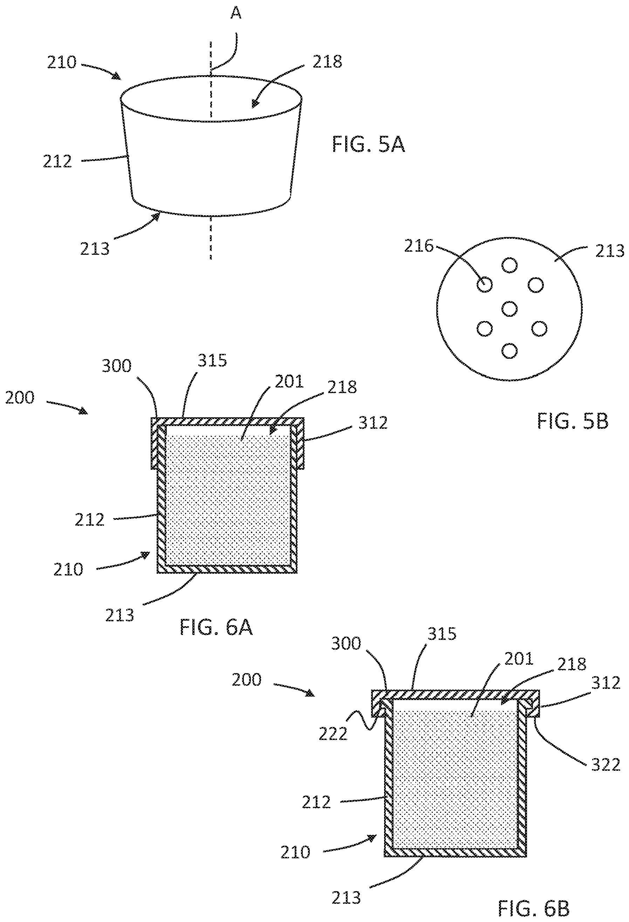

Referring now to **FIGS. 5A** and **5B****,** various aspects of the body **210** are shown. The body 210 may include a side wall **212** and a bottom wall **213** defining a cavity **218.** The side wall **212** may be cylindrical or frustoconical, as shown. The body **210** may have an open top. The body **210** may also include a plurality of apertures **216** on an opposite wall (for example, the bottom wall) to allow air flow through the cartridge when the cartridge is in use. An exemplary arrangement of apertures **216** at bottom wall **213** is shown in **FIG. 5B****.** The body **210** may define a center axis **A** extending through the body **210.**

**FIGS. 6A** and **6B** depict exemplary mechanism suitable for attaching the cap **300** to the body **210.** The cap **300** may be removably and replaceably engaged with or attached to the body **210.** The cap **300** may be attached by friction fit as shown in **FIG. 6A****.** The cap **300** may also be attached by snap fit as shown in **FIG. 6B****.** The body **210** may include a structure, such as a lip **222,** and the cap **300** may include a corresponding structure, such as lip **322,** that fits over the structure on the body **210** and holds the cap **300** rotatably in place. Other attachment mechanisms are also possible, such as bayonet connection and a threaded connection. Any of the attachment mechanisms described or mentioned here can be combined with any of the embodiments shown in **FIGS. 2A-4D****.**

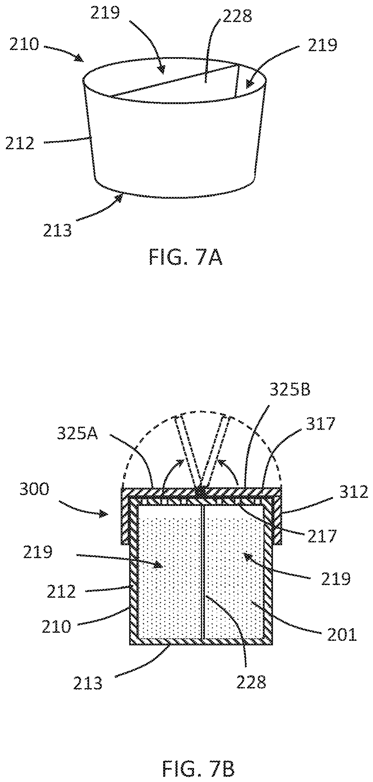

**FIG. 7A** shows a cartridge body **210** divided into compartments **219** by an interior wall **228.** The interior walls **228** may extend from the side wall **212** on one side to the side wall **212** on the opposite side. The cartridge body **210** may be combined, for example, with a cap **300** including a hatch **325.** **FIG. 7B** shows the cartridge body **210** of **FIG. 7A** combined with a cap **300** that includes a first hatch **325A** and a second hatch **325B.** The hatches **325A, 325B** may be aligned with the compartments **219** of the body such that a user may selectively open and close a compartment **219.** Opening the hatch **325A** or **325 B** allows airflow from openings at the bottom **213** (not shown) through the open compartment **219** and through the openings **217** at the top wall **215** of the body **210.**

## Claims

1. A cartridge (200) for housing an aerosol-forming substrate (201), the cartridge comprising:
a body (210) comprising one or more walls and a top wall (215), and a body opening in the top wall;
a cavity (218) in the body in fluid communication with the body opening;
a cap (300) removably and replaceably engaged with the body and extending across the body opening, the cap comprising a cap opening (317), wherein by rotating the cap to a first position the cap opening is aligned with the body opening and by rotating the cap to a second position the cap opening is not aligned with the body opening such that the cap closes the cartridge; and
a removable layer (414) removably attached to the body and disposed between the body and the cap.

2. A cartridge according to claim 1, wherein the body opening comprises a hole (217) or a plurality of holes through the top wall.

3. A cartridge according to any of the preceding claims, wherein the cap opening comprises a plurality of holes.

4. A cartridge according to any of the preceding claims, wherein the removable layer removably seals the body opening.

5. A cartridge according to any of the preceding claims, wherein the body comprises a cylindrical body portion.

6. A cartridge according to any of the preceding claims, wherein the body comprises a frustoconical portion.

7. A cartridge according to claim 5, wherein the cap comprises a lid portion (315) comprising the cap opening, and a cylindrical cap portion extending from the lid portion, the cylindrical cap portion being constructed to mate with the cylindrical body portion.

8. A cartridge according to any of the preceding claims comprising an aerosol-forming substrate disposed in the cavity, preferably wherein the aerosol-forming substrate comprises one or both of tobacco and an aerosol-forming shisha substrate.

9. A shisha system comprising:
a cartridge comprising:
a body comprising one or more walls, the one or more walls comprising a wall opening;
a cavity in the body in fluid communication with the wall opening;
an aerosol-forming substrate disposed in the cavity; and
a cap removably and replaceably engaged with the body and covering the wall opening, the cap comprising a cap opening and a hatch (325) covering the cap opening; and
a shisha device (100) comprising:
a receptacle (140) for receiving the cartridge;
a heating element (160) for heating the aerosol-forming substrate when the cartridge is received in the receptacle of the shisha device;
a vessel (17) having a liquid fill level and defining a head space above the liquid fill level;
an aerosol conduit for conveying aerosol from the receptacle to the vessel; and
an outlet (15) in communication with the head space.

10. A shisha system according to claim 9, wherein the cap and the body are constructed to engage via mechanical engagement.

11. A shisha system according to claim 9 or 10, wherein the cap and the body are constructed to engage via a snap fit, a bayonet connection, or a threaded fit.

12. A shisha system according to any one of claims 9-11 further comprising a removable layer between the body and the cap, wherein the removable layer is removably attached to the body and removably seals the wall opening.

13. A shisha system according to any of claims 9-12 comprising an aerosol-forming substrate disposed in the cavity, preferably wherein the aerosol-forming substrate comprises tobacco.

14. A shisha system according to claim 13, wherein the aerosol-forming substrate comprises a shisha aerosol-forming substrate.

15. A shisha system according to any one of claims 9-14, wherein the body comprises one or more interior walls (228) that divide the cavity into compartments (219), and optionally wherein the cap comprises a second cap opening and a second hatch (325B) covering the second cap opening.

## Patentansprüche

1. Patrone (200) zur Aufnahme eines aerosolbildenden Substrats (201), die Patrone umfassend:
einen Körper (210), umfassend eine oder mehrere Wände und eine obere Wand (215), und eine Körperöffnung in der oberen Wand;
einen Hohlraum (218) in dem Körper in Fluidverbindung mit der Körperöffnung;
eine Kappe (300), die entfernbar und austauschbar mit dem Körper in Eingriff steht und sich über die Körperöffnung erstreckt, wobei die Kappe eine Kappenöffnung (317) aufweist, wobei durch Drehen der Kappe in eine erste Stellung die Kappenöffnung mit der Körperöffnung ausgerichtet ist und durch Drehen der Kappe in eine zweite Stellung die Kappenöffnung nicht mit der Körperöffnung ausgerichtet ist, sodass die Kappe die Patrone verschließt; und
eine entfernbare Schicht (414), die entfernbar an dem Körper befestigt und zwischen dem Körper und der Kappe angeordnet ist.

2. Patrone nach Anspruch 1, wobei die Körperöffnung ein Loch (217) oder eine Vielzahl von Löchern durch die obere Wand aufweist.

3. Patrone nach einem beliebigen der vorhergehenden Ansprüche, wobei die Kappenöffnung eine Vielzahl von Löchern aufweist.

4. Patrone nach einem beliebigen der vorhergehenden Ansprüche, wobei die entfernbare Schicht die Körperöffnung entfernbar abdichtet.

5. Patrone nach einem beliebigen der vorhergehenden Ansprüche, wobei der Körper einen zylindrischen Körperabschnitt aufweist.

6. Patrone nach einem der beliebigen vorhergehenden Ansprüche, wobei der Körper einen kegelstumpfförmigen Abschnitt aufweist.

7. Patrone nach Anspruch 5, wobei die Kappe einen die Kappenöffnung umfassenden Deckelabschnitt (315) und einen sich von dem Deckelabschnitt erstreckenden zylindrischen Kappenabschnitt aufweist, wobei der zylindrische Kappenabschnitt so konstruiert ist, dass er mit dem zylindrischen Körperabschnitt zusammenpasst.

8. Patrone nach einem beliebigen der vorhergehenden Ansprüche, umfassend ein in dem Hohlraum angeordnetes aerosolbildendes Substrat, wobei das aerosolbildende Substrat eines oder beide von Tabak und einem aerosolbildenden Shisha-Substrat umfasst.

9. Shisha-System, umfassend:
eine Patrone, umfassend:
einen Körper, aufweisend eine oder mehrere Wände, wobei die eine oder mehreren Wände eine Wandöffnung aufweisen;
einen Hohlraum in dem Körper in Fluidverbindung mit der Wandöffnung;
ein in dem Hohlraum angeordnetes aerosolbildendes Substrat;
und
eine Kappe, die entfernbar und austauschbar in Eingriff mit dem Körper steht und die Wandöffnung abdeckt, wobei die Kappe eine Kappenöffnung und eine Klappe (325) aufweist, die die Kappenöffnung abdeckt; und
eine Shisha-Vorrichtung (100), umfassend:
eine Aufnahme (140) zur Aufnahme der Patrone;
ein Heizelement (160) zum Erwärmen des aerosolbildenden Substrats, wenn die Patrone in der Aufnahme der Shisha-Vorrichtung aufgenommen ist;
ein Gefäß (17), aufweisend einen Flüssigkeitsfüllstand und einen Kopfraum oberhalb des Flüssigkeitsfüllstandes definierend;
eine Aerosolleitung zum Fördern von Aerosol aus der Aufnahme in das Gefäß; und
einen mit dem Kopfraum in Verbindung stehenden Auslass (15).

10. Shisha-System nach Anspruch 9, wobei die Kappe und der Körper ausgebildet sind, um durch mechanischen Eingriff in Eingriff zu stehen.

11. Shisha-System nach Anspruch 9 oder 10, wobei die Kappe und der Körper ausgebildet sind, um über einen Schnappverschluss, eine Bajonettverbindung oder eine Gewindepassung in Eingriff zu stehen.

12. Shisha-System nach einem der Ansprüche 9 bis 11, ferner umfassend eine entfernbare Schicht zwischen dem Körper und der Kappe, wobei die entfernbare Schicht entfernbar am Körper angebracht ist und die Wandöffnung entfernbar abdichtet.

13. Shisha-System nach einem der Ansprüche 9 bis 12, umfassend ein in dem Hohlraum angeordnetes aerosolbildendes Substrat, wobei das aerosolbildende Substrat Tabak umfasst.

14. Shisha-System nach Anspruch 13, wobei das aerosolbildende Substrat ein aerosolbildendes Shisha-Substrat umfasst.

15. Shisha-System nach einem der Ansprüche 9 bis 14, wobei der Körper eine oder mehrere Innenwände (228) aufweist, die den Hohlraum in Kammern (219) unterteilen, und optional, wobei die Kappe eine zweite Kappenöffnung und eine zweite Klappe (325B) aufweist, die die zweite Kappenöffnung abdeckt.

## Revendications

1. Cartouche (200) destinée à loger un substrat formant aérosol (201), la cartouche comprenant :
un corps (210) comprenant une ou plusieurs parois et une paroi de sommet (215), et une ouverture de corps dans la paroi de sommet ;
une cavité (218) dans le corps en communication fluidique avec l'ouverture de corps ;
un capuchon (300) en prise de manière amovible et remplaçable avec le corps et s'étendant sur l'ouverture de corps, le capuchon comprenant une ouverture de capuchon (317), dans laquelle par rotation du capuchon vers une première position l'ouverture de capuchon est alignée avec l'ouverture de corps et par rotation du capuchon vers une deuxième position l'ouverture de capuchon n'est pas alignée avec l'ouverture de corps de sorte que le capuchon ferme la cartouche ; et
une couche amovible (414) fixée de manière amovible au corps et disposée entre le corps et le capuchon.

2. Cartouche selon la revendication 1, dans laquelle l'ouverture de corps comprend un orifice (217) ou une pluralité d'orifices à travers la paroi de sommet.

3. Cartouche selon l'une quelconque des revendications précédentes, dans laquelle l'ouverture de capuchon comprend une pluralité d'orifices.

4. Cartouche selon l'une quelconque des revendications précédentes, dans laquelle la couche amovible scelle de manière amovible l'ouverture de corps.

5. Cartouche selon l'une quelconque des revendications précédentes, dans laquelle le corps comprend une portion de corps cylindrique.

6. Cartouche selon l'une quelconque des revendications précédentes, dans laquelle le corps comprend une portion tronconique.

7. Cartouche selon la revendication 5, dans laquelle le capuchon comprend une portion de couvercle (315) comprenant l'ouverture de capuchon, et une portion de capuchon cylindrique s'étendant depuis la portion de couvercle, la portion de capuchon cylindrique étant construite pour s'accoupler avec la portion de corps cylindrique.

8. Cartouche selon l'une quelconque des revendications précédentes, comprenant un substrat formant aérosol disposé dans la cavité, de préférence dans laquelle le substrat formant aérosol comprend l'un ou les deux parmi du tabac et un substrat à shisha formant aérosol.

9. Système à shisha comprenant :
une cartouche comprenant :
un corps comprenant une ou plusieurs parois, les une ou plusieurs parois comprenant une ouverture de paroi ;
une cavité dans le corps en communication fluidique avec l'ouverture de paroi ;
un substrat formant aérosol disposé dans la cavité ; et
un capuchon en prise de manière amovible et remplaçable avec le corps et couvrant l'ouverture de paroi, le capuchon comprenant une ouverture de capuchon et une trappe (325) couvrant l'ouverture de capuchon ; et
un dispositif à shisha (100) comprenant :
un réceptacle (140) destiné à recevoir la cartouche ;
un élément de chauffage (160) destiné à chauffer le substrat formant aérosol lorsque la cartouche est reçue dans le réceptacle du dispositif à shisha ;
un récipient (17) ayant un niveau de remplissage de liquide et définissant un espace de tête au-dessus du niveau de remplissage de liquide ;
un conduit d'aérosol destiné à transporter un aérosol depuis le réceptacle jusqu'au récipient ; et
une sortie (15) en communication avec l'espace de tête.

10. Système à shisha selon la revendication 9, dans lequel le capuchon et le corps sont construits pour se mettre en prise par une prise mécanique.

11. Système à shisha selon la revendication 9 ou 10, dans lequel le capuchon et le corps sont construits pour venir en prise par un ajustement par encliquetage, un raccord à baïonnette ou un ajustement fileté.

12. Système à shisha selon l'une quelconque des revendications 9 à 11, comprenant en outre une couche amovible entre le corps et le capuchon, dans lequel la couche amovible est fixée de manière amovible au corps et scelle de manière amovible l'ouverture de paroi.

13. Système à shisha selon l'une quelconque des revendications 9 à 12, comprenant un substrat formant aérosol disposé dans la cavité, de préférence dans lequel le substrat formant aérosol comprend du tabac.

14. Système à shisha selon la revendication 13, dans lequel le substrat formant aérosol comprend un substrat à shisha formant aérosol.

15. Système à shisha selon l'une quelconque des revendications 9 à 14, dans lequel le corps comprend une ou plusieurs parois intérieures (228) qui divisent la cavité en compartiments (219), et facultativement dans lequel le capuchon comprend une deuxième ouverture de capuchon et une deuxième trappe (325B) couvrant la deuxième ouverture de capuchon.
